(19) Europäisches Patentamt
European Patent Office
Office européen des brevets

(11) **EP 4 494 578 A1**

(12) **EUROPEAN PATENT APPLICATION**

(43) Date of publication:
**22.01.2025 Bulletin 2025/04**

(21) Application number: **24189201.7**

(22) Date of filing: **17.07.2024**

(51) International Patent Classification (IPC):
**A61B 17/32** $^{(2006.01)}$ **A61B 18/12** $^{(2006.01)}$

(52) Cooperative Patent Classification (CPC):
**A61B 17/320068; A61B 18/1445;** A61B 18/1206;
A61B 2017/00017

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **20.07.2023 US 202363527886 P**

(71) Applicant: **Olympus Winter & Ibe GmbH
22045 Hamburg (DE)**

(72) Inventors:
• **Dijkstra, Jelle
12205 Berlin (DE)**
• **Fähsing, Thomas
12305 Berlin (DE)**

(74) Representative: **Eisenführ Speiser
Patentanwälte Rechtsanwälte PartGmbB
Postfach 10 60 78
28060 Bremen (DE)**

(54) **ELECTROSURGICAL GENERATOR FOR CONTROLLING AN ULTRASONIC TRANSDUCER OF AN ELECTROSURGICAL INSTRUMENT**

(57)     The invention relates to a method for controlling an ultrasonic transducer (206) of an electrosurgical instrument (102, 202), the ultrasonic transducer (206) is driven by an alternating electrical overall current signal ($i_m$), having a signal frequency (f), driving the overall current signal ($i_m$) results in an alternating electrical instrument voltage signal ($u_m$) of the transducer (206), depending on a mechanical oscillation of the transducer (206), the instrument voltage signal ($u_m$) having a transducer phase shift with respect to a main current component of the overall current signal ($i_m$) depending on the mechanical oscillations of the transducer, the ultrasonic transducer (206) is having a fluctuating mechanical resonance frequency, an amplifier (204) is controlled to provide the electrical overall current signal ($i_m$) and adjust the signal frequency (f) towards the resonance frequency depending on the transducer phase shift, and for determining the transducer phase shift, calculating signal coefficients for the main current component and the instrument voltage signal ($u_m$), the signal coefficients being representative for a phase relationship of the main current component or the instrument voltage signal respectively with respect to a common reference signal, and calculating from the signal coefficients the transducer phase shift.

Fig. 2

EP 4 494 578 A1

**Description**

The present invention is directed to a method for controlling an ultrasonic transducer of an electrosurgical instrument. The invention is also directed to an electrosurgical generator for controlling an ultrasonic transducer of an electrosurgical instrument. The invention is also directed to an electrosurgical installation comprising an electrosurgical generator and an ultrasonic transducer of an electrosurgical instrument connected to the electrosurgical generator.

Ultrasonic transducers are used to transform an alternating electrical signal into a mechanical oscillation of an electrosurgical instrument having an ultrasonic frequency. In order to do that, the alternating electrical signal is basically stimulating the mechanical oscillation at a resonance frequency of the ultrasonic transducers.

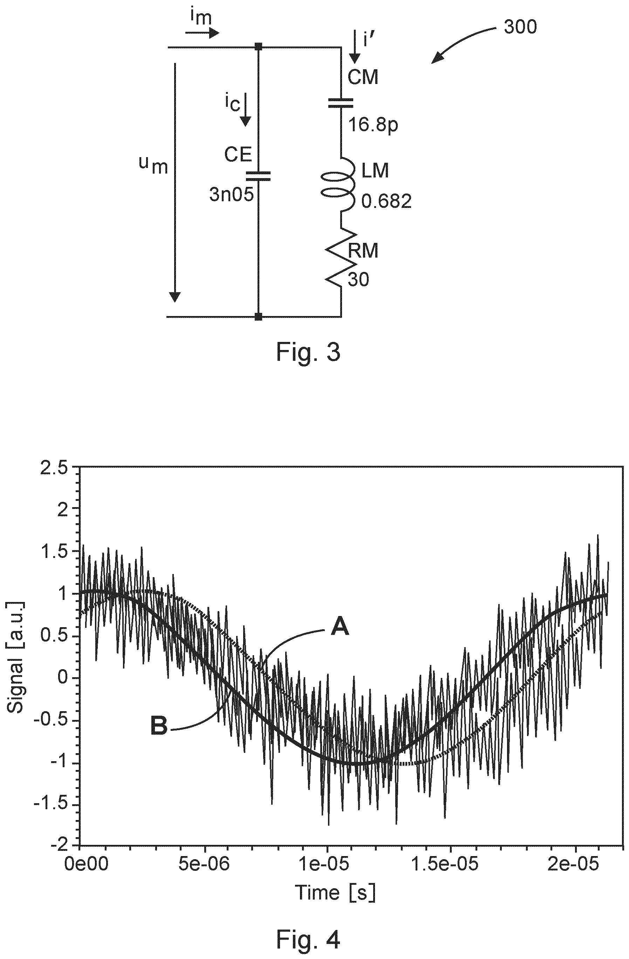

Ultrasonic transducers can be modelled according to an equivalent circuit shown in Figure 3. The model shows a mechanical resonance circuit as an electrical equivalent (CM + LM + RM) and the parallel electrical capacitance (CE).

Ultrasonic transducers need to be driven at their mechanical resonance frequency. Since the resonance frequency changes with mechanical load, a control loop may be required to track the changing resonance frequency. A PLL can be used to adjust the frequency according to the measured phase shift between the voltage across the transducer and the current of the electrical equivalent of the mechanical resonance circuit, which can be understood as the mechanical current. When the phase shift is positive, the resonance circuit is inductive, and the frequency is too high. When the phase shift is negative, the resonance circuit is capacitive, and the frequency is too low.

A possible control loop may contain analogue sensors, ADCs and a digital controller (FPGA) that drives the amplifier with a calculated frequency. The controller may determine the phase shift between current and voltage by comparing their respective zero-crossings in the time domain.

Since zero-crossing detection is very sensitive to noise, the current and voltage signals must be filtered very carefully using a low-pass filter.

Filtering the signals in the digital domain is challenging. On the one hand, the signals must be oversampled by a high factor to accurately determine the instance where the signal crosses zero. On the other hand, a robust FIR low-pass filter requires many taps when the cut-off frequency is low compared to sampling frequency.

One possibility to solve this problem is by filtering the signals in the analog domain. This introduces the problem that filters produce a phase shift of the signals. Therefore, both analog filter paths need to be matched very closely to make sure that no additional relative phase shift is introduced. This requires complex filter structures with tightly tolerated components.

Accordingly, an object of the present invention is to address at least one of the above-mentioned problems. In particular, a solution shall be provided for improving the control of an ultrasonic transducer, in particular a solution shall be provided for identifying the mechanical resonance frequency of ultrasonic transducers in an improved manner and/or made more reliable. At least a solution shall be suggested which is an alternative solution with respect to known solutions.

According to the invention, a method is suggested according to claim 1. Thus, a method for controlling an ultrasonic transducer of an electrosurgical instrument is suggested. Such ultrasonic transducer is driven by an alternating electrical overall current signal having a signal frequency. Such transducer may be driven mainly by means of a current source having a voltage limit.

Such overall current signal is resulting in an alternating electrical instrument voltage signal of the transducer, depending on a mechanical oscillation of the transducer. In other words, the alternating electrical overall current signal, which can be a mainly sinusoidal current signal, is applied to the transducer. Depending on the transducer, the instrument voltage signal results. The shape of the instrument voltage signal mainly depends on a mechanical oscillation of the transducer.

The instrument voltage signal has a transducer phase shift with respect to a main current component of the overall current signal depending on the mechanical oscillations of the transducer. Thus, depending on the mechanical oscillations of the transducer, there will be a resulting phase shift, i.e. a phase shift between the instrument voltage signal and the main current component.

The ultrasonic transducer is having a fluctuating mechanical resonance frequency. Such mechanical resonance frequency might be in a range of 45 kHz - 50 kHz, in particular in the area of 46 kHz - 49 kHz. The resonance frequency may vary within this range. Of course it could also be outside of this range. However, the mechanical resonance frequency is fluctuating, at least slightly, and that may depend on the use of the electrosurgical instrument. For example, the resonance frequency may be different depending on whether the electrosurgical instrument is in contact with tissue or not.

There is also an amplifier controlled to provide the electrical overall current signal and adjust the signal frequency towards the resonance frequency depending on the transducer phase shift. Accordingly, the transducer phase shift indicates if and possibly how well the mechanical resonance frequency is met by the voltage signal and current signal. The resonance frequency is met, if there is no phase shift. Therefore, it is important to determine the transducer phase shift and adjust the signal frequency such that the amplitude of the phase shift decreases. In particular, a positive phase shift indicates that the resonance circuit is inductive and the frequency is too high and thus shall be reduced. On the other hand, when the phase shift is negative, the resonance circuit is capacitive, the frequency is too low and should be increased.

For determining the transducer phase shift, calculating signal coefficients for the current signal and the main voltage signal is suggested. Such signal coefficients shall be representative for a phase relationship of the main current component or the instrument voltage signal, respectively, with respect to a common reference signal. Accordingly, there are calculated signal coefficients for the main current component which are representative for a phase relationship of the main current component with respect to a common reference signal and there are signal coefficients for the instrument voltage signal calculated which are representative for a phase relationship of the instrument voltage signal with respect to the common reference signal.

Such common reference signal may be predetermined for the purpose of comparison. However, such common reference signal may also just be a theoretical or virtual reference signal. As will be explained further below, a calculation shall be used for calculating said signal coefficients for the main current component and also for the instrument voltage signal, using the same algorithm and using sampled values of a current signal and the instrument voltage signal, respectively, for the same time interval. In this way, using the same time interval, there are the same boundary conditions for calculating the signal coefficients for both signals resulting in having the same common reference signal. Accordingly, the relationship regarding the transducer phase shift can be identified when for both signals the same common reference signal is considered or is at least underlying the calculation without explicitly giving this common reference signal.

It is further suggested calculating from the signal coefficients the transducer phase shift.

Accordingly, the signal coefficients can be calculated characterizing the particular signal also with respect to their phase relationship with respect to the common reference signal and such phase relationship can also be denoted as phase angle. Accordingly, these signal coefficients also specify the phase angle of both signals and in this way the transducer phase shift can be calculated, in particular by subtracting the one phase angle from the other. In this way, the transducer phase shift can be calculated in an easy and reliable manner without comparing any signal waveforms of the measured signals. It can also be done without identifying a zero crossing, or other significant point of the two signals.

In particular, for calculating the signal coefficients, sampled values of both signals for a full period or at least for a half period are taken into account. Accordingly, the result is in particular two coefficients for each signal, thus in total four coefficients, which are calculated based on many sampled values. For example, each signal may be measured for one period using 100 samples each, maybe more. With respect to this example, two coefficients of one sampled signal are calculated based on 100 samples.

In this way, problems of any noise of the signals can be at least reduced.

According to one aspect, it is suggested that depending on the calculated transducer phase shift the signal frequency is adjusted to match the mechanical resonance frequency or to get closer to it. Accordingly, the transducer phase shift is calculated and it indicates a deviation between the mechanical resonance frequency and the signal frequency. Therefore, the signal frequency is adjusted to match the mechanical resonance frequency, at least to better match the mechanical resonance frequency. It was found that it is not necessary to identify the mechanical resonance frequency, but only to identify whether the signal frequency deviates from it.

It is in particular suggested to determine whether the signal frequency is above or below the resonance frequency and then raise or reduce the signal frequency accordingly to get closer to the resonance frequency.

Raising or reducing the signal frequency to get closer to the resonance frequency can be done in different ways. One possibility is to raise or reduce the signal frequency by a constant value. Such discrete way is in fact almost a continuous approach as there is a high repetition frequency for performing such raising or reducing. Thus, a difference between the signal frequency and the resonance frequency leads to a change, in particular small change of the signal frequency letting it get at least a little bit closer to the mechanical resonance frequency. This step is continuously repeated including repeating the calculating of the transducer phase shift. Accordingly, such step of adjusting is repeated quite often and each time the signal frequency gets a little bit closer to the mechanical resonance frequency, until it is almost reached.

Another possibility is to use a feedback control loop having a PID, or PI-control. According to the integral part of the control, the signal frequency found will keep its value when the input is zero.

There are thus different possibilities to design such feedback control loop. One is to use PI or PID-control and another is to increase by a constant value. In both cases, the feedback can be the calculated transducer phase shift or a calculated difference between the signal frequency and a calculated, at least roughly calculated, mechanical resonance frequency. In both cases, the input value tends to zero with the signal frequency matching the mechanical resonance frequency. It was found that it is easiest to just feedback the calculated transducer phase shift and just to use a proportional factor in order to transfer the transducer phase shift to a frequency difference between the signal frequency and the mechanical resonance frequency. Such proportional factor can be calculated or determined in a simulation or determined by measurements during a test operation, when the mechanical resonance frequency is measured or estimated.

However, it is also possible and suggested as one aspect to calculate an actual value of the mechanical resonance frequency depending on the calculated transducer phase shift and to set the signal frequency depending on the calculated mechanical resonance frequency, i.e. set the signal frequency to the mechanical resonance frequency. It was found that the calculated transducer phase shift can be correlated to a frequency difference between the signal frequency and the mechanical resonance frequency. As the signal frequency is known, as the alternating electric overall current signal is generated, the mechanical resonance frequency can thus quite precisely be calculated.

It was found that the variation of the mechanical resonance frequency is not high and in particular below 10% of a mean mechanical resonance frequency, possibly even below 5% of such mean mechanical resonance frequency. Accordingly, it can be assumed that the calculated transducer phase shift is almost proportional to the difference between the mechanical resonance frequency and the signal frequency. Therefore, the mechanical resonance frequency can quite easily and quite precisely be calculated in this way.

According to one aspect, it is suggested that the calculating of the signal coefficients is based on samples of the overall current signal and samples of the instrument voltage signal. In this way, these values can be sampled during a control cycle and each time new values can be calculated, a new transducer phase shift can be calculated and the signal frequency can be adapted accordingly.

Accordingly, the instrument voltage signal will be measured and the overall current signal may also be measured. However, the overall current signal may also just be known as it is generated as part of this the method, in particular by an electrosurgical generator generating such current signal.

Based on such sampled values, a control cycle can be implemented calculating the transducer phase shift for each sample time and thus also adjusting the signal frequency at each sample time.

According to one aspect, the signal coefficients characterize the first harmonic of the corresponding signal. I.e. two signal coefficients of the main current component characterize the first harmonic of the main current component and two coefficients of the instrument voltage signal characterize the first harmonic of the instrument voltage signal. Such coefficients can be calculated in a quasi-continuous way using a fast Fourier transform.

In this way, for each control cycle and thus for each sample time it is possible to calculate updated signal coefficients for the main current component and the instrument voltage signal and based on that calculate the transducer phase shift.

In this way, a reliable way of calculating is provided avoiding to observe any zero-crossings of the particular signals, which is otherwise often used to calculate a particular frequency and a particular phase angle.

According to one aspect, it is suggested that for each signal, i.e. for the main current component and the instrument voltage signal, the signal coefficients are the first harmonic Fourier trigonometric coefficients $a_1$ and $b_1$ calculated according to the formula

$$a_1 = \frac{2}{N} \sum_{k=0}^{N-1} x[k] * \cos\left(2\pi \frac{k}{N}\right)$$

$$b_1 = \frac{2}{N} \sum_{k=0}^{N-1} x[k] * \sin\left(2\pi \frac{k}{N}\right)$$

wherein

- $x[k]$ are samples of the corresponding signal and
- $N$ is the number of samples per signal, and wherein
- the samples are taken over a predetermined time interval and/or a time interval of one period corresponding to a nominal value of the resonance frequency.

This way of calculating the signal coefficients also uses samples of the corresponding signal, i.e. samples of the current signal and samples of the main voltage signal. The variable k can also be understood as the discrete sample time and the samples from k=0 to k = N-1 can reflect the samples of one period of the signal frequency.

Accordingly, it was also found that the signal frequency is well-known, as the overall current signal is generated and thus its frequency is known. Accordingly, the period of such alternating, i.e. mainly sinusoidal signal, is also well-known. Therefore, said formula for calculating the signal coefficients is applied to sampled signals of exactly one signal period leading to a quite precise result of calculating this first harmonic Fourier trigonometric coefficients $a_1$ and $b_1$.

Of course, it is also possible to use samples of two or more whole number of signal periods.

Even for such calculation using a complete signal period a fast calculation and thus fast control is still possible, as the underlying signal frequency is expected to be in the range of 40 kHz and 50 kHz. It may also be a bit higher or a bit lower. However, as the signal frequency is this high, the time needed to sample values of one signal period is in the range of approximately 20 μs. It was also found that taking samples over one signal period is enough as it is enough to change the frequency of the current signal per signal period.

According to one aspect, it is suggested that for the signal coefficients for each signal being the first harmonic

Fourier trigonometric coefficients $a_1$ and $b_1$, the absolute phase shift ($\varphi_a$ for each signal is calculated based on the formula $\varphi_a = atan2(a_1,b_1)$) and the transducer phase shift is calculated as a difference of the absolute phase shifts of both signals. I.e., the transducer phase shift is calculated as the difference of the absolute phase shifts of the main current component and the instrument voltage signal.

Accordingly, there is a quite adequate calculation of the absolute phase shifts and thus the transducer phase shift which provides good results and is not depending on observing any zero-crossings. Such calculation is also tolerant to some noise and other distortions of the corresponding signal, i.e. of the current signal or main voltage signal.

According to one aspect, it is suggested that

- for the signal coefficients for each signal being the first harmonic Fourier trigonometric coefficients $a_1$ and $b_1$
- the transducer phase shift is calculated by an approximation such that the transducer phase shift $\varphi_t$ is calculated as the difference of the quotient $a_1/b_1$ of both signals, in particular $\varphi_t$ is calculated using the formula

$$\varphi_t = a_{1,i}/b_{1,i} - a_{1,u}/b_{1,u}$$

with

$a_{1,i}$ and $b_{1,i}$ being the first harmonic Fourier trigonometric coefficients $a_1$ and $b_1$ of the main current component and

$a_{1,u}$ and $b_{1,u}$ being the first harmonic Fourier trigonometric coefficients $a_1$ and $b_1$ of the instrument voltage signal.

Accordingly, the transducer phase shift can be calculated in an easy way and the use of the trigonometric calculation atan2 can be avoided. It was found that the transducer phase shift, i.e. said corresponding phase shift angle, is expected to be quite small, i.e. much below 30°, that such approximation is feasible. It was also found that according to the suggested adjusting of the signal frequency towards the mechanical resonance frequency, it is possible by such control to take care of having a good match of the signal frequency to the mechanical resonance frequency and thus have a small transducer phase shift. Accordingly, the suggested approximation can be used.

It was also found that the calculation of the transducer phase shift is only needed to adjust the signal frequency towards the mechanical resonance frequency. Accordingly, any inaccuracy of the calculated transducer phase shift is not or not much influencing the control of adjusting the signal frequency. In particular, inaccuracies are only expected for large transducer phase shifts. But at large transducer phase shifts inaccuracies of the adjusting of the signal frequency are acceptable. A higher accuracy is needed when the signal frequency is close to match the mechanical resonance frequency. However, at this stage, the inaccuracy according to the approximation is also quite small resulting in a fairly small control inaccuracy if at all noticeable.

According to one aspect, it is suggested that calculating the transducer phase shift and/or adjusting the signal frequency to match the mechanical resonance frequency or to get closer to it is repeated regularly for tracking changes of the mechanical resonance frequency, and in particular the calculating or adjusting is repeated with a predetermined repetition frequency being at least one repetition per 10 signal periods, preferably at least one repetition per 2 signal periods, in particular one repetition per signal period.

It was found that by this permanent repetition the signal frequency can be controlled to be very close to the mechanical resonance frequency. By repeating the calculation of the transducer phase shift and/or the adjusting of the signal frequency, the ultrasonic transducer and thus the corresponding electrosurgical instrument can almost always be operated effectively.

The mechanical resonance frequency may depend on the electrosurgical instrument being in touch with tissue or other material or not being in touch with any material. Accordingly, just a small movement of the electrosurgical instrument when in operation may change said situation and accordingly the mechanical resonance frequency may also change. However, repeating the calculating and/or adjusting with one repetition per signal period leads to a fast tracking of the changed situation, as the signal period is approximately in the range of 20 μs. Compared to that, a manual movement of the electrosurgical instrument when in operation can be quite slow when compared to said high repetition frequency.

Therefore, the change of such situation can be tracked quickly enough to keep the electrosurgical instrument in good operation. As explained above, such ultrasonic transducer and thus such ultrasonic electrosurgical instrument which is used as the electrosurgical instrument only operates well if the overall current signal and the resulting alternating electrical voltage signal almost match the mechanical resonance frequency. By this quick repetition of the calculating and/or adjusting such good operation can be ensured.

Of course, it would also be possible to slow the repetition frequency down to one repetition per 10 signal periods, or a value in between this one repetition per 10 signal periods and the one repetition per 1 signal period.

According to one aspect, it is suggested that the signal coefficients, in particular the signal coefficients for each signal being the first harmonic Fourier trigonometric coefficients $a_1$ and $b_1$, are used to calculate an rms-value of the current signal, in particular of the main current component, and/or an rms-value of the voltage signal and/or a real power value of both signals. Accordingly, each rms-value and/or the real power value of both signals is calculated based on the first harmonic of the current signal and/or the voltage signal.

This is advantageous as there is a clear relationship between these frequencies according to the first harmonic and the deflection of the ultrasonic transducer and/or the electrosurgical instrument being an ultrasonic electrosurgical instrument. Accordingly, this part of the signal corresponding to the mechanical deflection is thus also the characteristic signal for considering this deflection. Therefore, considering such rms-values and/or the real power value provides the best relationship between the considered electrical signals and the mechanical behavior.

According to one aspect, it is suggested that

- the overall current signal, having a main current component and a capacitive current component, the capacitive current component, depending on a capacitance of the transducer, and

- the main current component is calculated by subtracting the capacitive current component from the overall current signal, in particular from a measured alternating electrical current signal of the transducer, and/or

- first harmonic Fourier trigonometric coefficients $a_1$ and $b_1$ are used for calculating the capacitive current signal, wherein in particular

- the capacitive current component ic is calculated for the capacitance C and the frequency $\omega$ using the formula:

$$i_C = C*\omega*a_1*\cos(\omega*t) \text{ or } i_C = -C*\omega*b_1*\sin(\omega*t).$$

The nominal frequency $\omega_0$ can be used for $\omega$. That is relevant for any formula discussed.

Accordingly, it was found that the overall current, which is in particular the current that is measured, has a main current component and a capacitive current component. Hence, basically there is the main current component and the capacitive current component as an additional component. This capacitive current component depends on a capacitance of the transducer. This capacitive current component is basically - simply speaking - not involved in driving the mechanical movement of the ultrasonic electrosurgical instrument.

However, such capacitive current component results in a deviation between the phase angle of the voltage signal and the overall current signal which is not indicating any deviation between the signal frequency and the mechanical resonance frequency. Only the deviation of the phase angle between the main current component and the instrument voltage signal indicates the deviation between the signal frequency and the mechanical resonance frequency. Accordingly, the overall current signal is cleared from this capacitive current component.

Accordingly, the main current component is calculated by subtracting the capacitive current component from the overall current signal. The overall current signal can be measured and thus the measured alternating electrical current signal can be used as the overall current signal. Of course, it might be possible that by generating this overall current signal the properties of this overall current signal might be well-known and these properties could be used instead of measuring the alternating electrical current signal, i.e. the overall current signal.

With the known capacitance of the ultrasonic transducer the capacitive current component can be calculated. One way of doing this is to use the first harmonic Fourier trigonometric coefficients $a_1$ and $b_1$. These can be calculated as explained above. In this way, basically only the first harmonic of the current signal is considered which is advantageous as basically only this first harmonic component influences the phase shift caused by the capacity. In addition, using the first harmonic Fourier trigonometric coefficients is reliable and tolerant to noise and distortions on the signal. In particular, the given formula provides a simple way to calculate the capacitive current component.

According to a further aspect, it is suggested that

- for calculating the transducer capacitance,

- the overall current signal is generated having a test frequency being different to the nominal value of the resonance frequency at least by a minimum frequency difference, resulting in an instrument voltage signal,

- the signal coefficients are calculated based on the overall current signal and the resulting instrument voltage signal having the test frequency, and

- the transducer capacitance is calculated based on these signal coefficients, wherein in particular

- the minimum frequency difference is at least 10kHz, in particular at least 20 kHz.

Accordingly, it was found that the main current component driving the mechanical oscillation of the transducer and thus the electrosurgical instrument is not very dominant when the signal frequency is very different from the mechanical resonance frequency. In this case, which is reached by having this minimum frequency difference of at least 10 kHz, the capacity is basically the only characteristic of the transducer influencing the overall current signal that is driven through the transducer. Accordingly, the relationship between the overall current signal and the resulting instrument voltage signal gives a clear indication of the capacitance.

Underlying the choice of a minimum frequency of at least 10 kHz is the understanding that the resonance frequency is in a range of about 40 kHz to 50 kHz. Accordingly, this minimum frequency of 10 kHz is a significant frequency difference resulting in almost not at all triggering any mechanical oscillation of the transducer or electrosurgical instrument. The minimum frequency could also be at least 20 kHz. In this way, the frequency is even further away from the mechanical resonance frequency. However, it should be kept in mind that the test frequency should at least not deviate too much, as in case of a too high deviation any nonlinearities of the capacitance can possibly not be neglected anymore. In that case, an inaccuracy of calculating the capacitance could be a result.

According to one aspect, it is suggested that

- for calculating the signal coefficients, in particular for calculating the signal coefficients of the instrument voltage signal,

- the instrument voltage signal is modified into a modified voltage signal by adding a virtual voltage signal, wherein

- the virtual voltage signal is calculated by multiplying the main current component with a predetermined virtual resistance, and in particular

- as the main current component a previously calculated main current component is used and,

- the previously calculated main current signal is calculated based on the signal coefficients previously calculated for the voltage signal and for the main current signal and/or

- the modified voltage signal is calculated directly using the signal coefficients previously calculated for the voltage signal and for the main current signal and/or

- coefficients of the modified voltage signal are calculated directly using signal coefficients previously calculated for the voltage signal and for the main current signal and using the predetermined virtual resistance.

The underlying idea of this aspect is that the measured voltage might have a very small amplitude in particular in view of noise which is often present on measured voltage signals. Thus, the evaluation or calculation might lead to problems, such as stability problems if noise level is too high, in relation to the signal amplitude.

It was found that such situation may occur if the instrument is in operation and operating at or close to the resonance frequency. In that case such virtual voltage signal, which is not suffering from noise, is added to the measured voltage signal and the result is a signal having an amplitude being high enough when compared with the noise amplitude.

As this added virtual signal is not exactly reflecting the actual voltage at the transducer, it might have a slightly different phase angle and that might result in a slight change of the determined value of the frequency. That might result in a small frequency deviation from the ideal resonance frequency, but such deviation is acceptable. It was also found that because of the frequent repetition of setting the frequency, only a small fluctuation about the ideal value of the frequency will result. That was found to be acceptable as depending on the load of the transducer the ideal resonance frequency will fluctuate also and thus the frequency must be adjusted quite often.

However, it was also found that when the frequency is not too close to the resonance frequency, the measured voltage is also higher and high enough to be measured without problems, i.e. high enough to be distinguished from the noise level. Accordingly, the virtual resistance can be chosen such, that the virtual voltage signal is only significant when the frequency is at or close to the resonance frequency. It is not significantly high, i.e. lower than the measured voltage signal, when the signal frequency deviates significantly from the resonance frequency.

Accordingly, when the signal frequency deviates significantly from the resonance frequency, the influence of the virtual resistance and/or the resulting virtual voltage can be neglected and accordingly the deviation from the resonance frequency can be detected with high precision enabling the operating point to move towards the resonance frequency.

Preferably the virtual voltage signal is chosen such, that its average rms-value is between 3 to 20 times, in particular 5 to 10 times the rms average value of the voltage signal at the resonance frequency. Based on known average values of the voltage at the resonance frequency and corresponding main current component, the virtual resistance can be calculated accordingly. The virtual resistance may be in a range of about 100-200 Ohms. It could also be chosen based on experiences.

For calculating the main current component, the following formula can be used, which is explained further below:

$$i'(t) \approx \left(a_{1,i} - CE \cdot \omega_0 \cdot b_{1,u}\right)\cos(\omega_0 t) + \left(b_{1,i} + CE \cdot \omega_0 \cdot a_{1,u}\right)\sin(\omega_0 t)$$

However, based on that it is also possible to directly calculate the modified voltage, i.e. the modified voltage signal using the signal coefficients previously calculated for the voltage signal and for the main current signal using the formula, which is explained further below:

$$u'(t) \approx \left(a_{1,u} + R_{virt}\left(a_{1,i} - CE \cdot \omega_0 \cdot b_{1,u}\right)\right)\cdot \cos\omega_0 t + \left(b_{1,u} + \left(b_{1,i} + CE \cdot \omega_0 \cdot a_{1,u}\right)\right)\sin\omega_0 t$$

This way, even the modified voltage signal can be calculated quite easily, just using these coefficients.

For the aspect that coefficients of the modified, and compensated, voltage signal $a_{1,u}'$ and $b_{1,u}'$ are calculated directly using signal coefficients previously calculated for the voltage signal and for the main current signal and using the predetermined virtual resistance, the following relationship can be used which is also explained below in more detail:

$$u'(t) \quad \leftrightarrow \quad a_{1,u} + R_{virt}\left(a_{1,i} - CE\omega_0 b_{1,u}\right) \ , \ \ b_{1,u} + R_{virt}\left(b_{1,i} + CE\omega_0 a_{1,u}\right)$$

Accordingly $a_{1,u}'$ and $b_{1,u}'$ can be calculated as follows:

$$\mathsf{a_{1,u}}' = a_{1,u} + R_{virt}\left(a_{1,i} - CE\omega_0 b_{1,u}\right)$$

$$\mathsf{b_{1,u}}'= b_{1,u} + R_{virt}\left(b_{1,i} + CE\omega_0 a_{1,u}\right)$$

According to one aspect, it is also suggested an electrosurgical generator for controlling an ultrasonic transducer of an electrosurgical instrument adapted to execute a method according to any of the preceding aspects.

Thus, the electrosurgical generator may have any corresponding control unit such as a digital controller, e.g. an FPGA and an amplifier connected to the controller and driven by the controller. The controller may provide a calculated frequency that will be submitted to the amplifier and the amplifier will then generate a corresponding current signal. The controller determines the phase shift between current and voltage and will base on that determination control the amplifier.

Accordingly, any method according to the explained aspects can be implemented at least partially as a computer program on such controller.

The electrosurgical generator may also comprise current and voltage sensors for measuring an overall current signal generated by the amplifier and for measuring a resulting instrument voltage signal. Such sensors may particularly be connected to the controller and/or to the amplifier.

There is also suggested an electrosurgical installation comprising an electrosurgical generator as explained above and an electrosurgical instrument. The electrosurgical instrument is connected to the electrosurgical generator and the electrosurgical instrument may have an ultrasonic transducer. In particular, the amplifier of the electrosurgical generator is connected to the ultrasonic transducer and provides an overall current signal to the ultrasonic transducer.

The invention will be explained by way of example based on the accompanying figures in more detail.

Figure 1    shows an electrosurgical generator having an electrosurgical instrument connected.

Figure 2    shows a simplified control structure.

Figure 3    shows an equivalent circuit of an ultrasonic transducer.

Figure 4    shows two sin signals reconstructed by calculating first order Fourier coefficients.

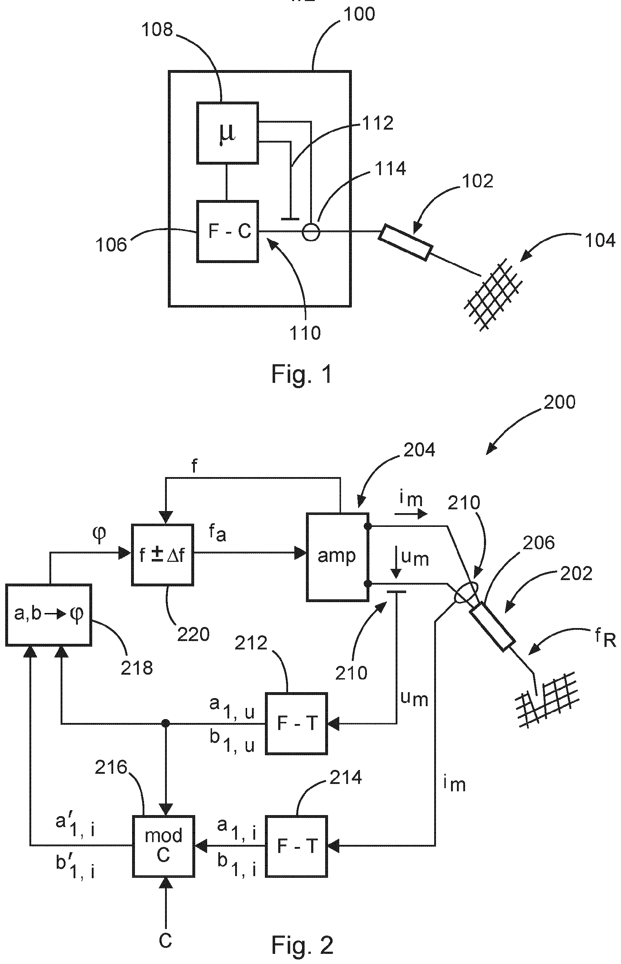

Figure 1 shows an electrosurgical generator 100 to which an electrosurgical instrument 102 is connected operating on a tissue 104 just schematically illustrated.

The electrosurgical generator 100 comprises a frequency converter 106 which is controlled by a control device 108. The frequency converter when in operation provides at its output 110 an overall current signal which is applied to the attached electrosurgical instrument 102, resulting in an instrument voltage. The instrument voltage is a simplified term for the instrument voltage signal and is thus a synonym for it.

There is a voltage sensor 112 for measuring the voltage signal and a current sensor 114 for measuring the overall current signal. These sensors 112, 114 are connected to the control device 108 for further consideration.

The control device 108 calculates from the current sample values provided by the current sensor 114 signal coefficients. Based on that, the control device 108 controls the frequency converter 106. Accordingly, at the frequency converter output, the overall current signal is generated depending on the calculated coefficients.

It is also possible that the voltage sensor 112 and/or the current sensor 114 are connected to the frequency converter 106 directly. It is also possible that the control device 108 is part of the frequency converter 106.

Figure 2 shows a simplified control structure 200 for controlling an ultrasonic transducer of an electrosurgical instrument. For this purpose, Figure 2 shows schematically such electrosurgical instrument 202 having an ultrasonic transducer. The electrosurgical instrument 202 and thus its ultrasonic transducer is connected to an amplifier 204. The amplifier 204 can be identical to the frequency converter 106 of Figure 1, or similar, or can be part of such frequency converter.

The amplifier 204 provides an alternating electrical overall current $i_m$ which is thus driven into the ultrasonic transducer 206 of the electrosurgical instrument 202. The overall current is a simplified term for the overall current signal and is thus a synonym for it. Driving this overall current $i_m$ results in an instrument voltage $u_m$. The overall current $i_m$ and accordingly the instrument voltage $u_m$ have a signal frequency f. The transducer 206 works best when this signal frequency f matches a mechanical resonance frequency of the transducer 206. To ensure that, the overall current $i_m$ and the instrument voltage $u_m$ are measured with a voltage sensor 208 and a current sensor 210. Accordingly, the instrument voltage $u_m$ and the overall current $i_m$ are measured. For simplification it is assumed that the overall current and its measured value are the same and that the instrument voltage and its measured value are also the same.

The measured instrument voltage $u_m$ is given to a voltage Fourier transform block 212 calculating the first harmonic Fourier trigonometric coefficients $a_{1,u}$ and $b_{1,u}$ of the voltage signal. Similarly, the measured overall current $i_m$ is given to a current Fourier transform block 214 calculating the first harmonic trigonometric coefficients $a_{1,i}$ and $b_{1,i}$ of the current signal.

Accordingly, the first Fourier trigonometric coefficients $a_{1,i}$ and $b_{1,i}$ of the measured overall current $i_m$ are representing the overall current $i_m$. However, for calculating the phase shift $\varphi$ which is relevant for identifying a deviation of the signal frequency f from the mechanical resonance frequency $f_R$ may be cleared by a capacitive current component. This capacitive current component can be calculated using the first harmonic Fourier trigonometric coefficients $a_{1,u}$ and $b_{1,u}$ of the measured instrument voltage $u_m$ and using the known capacitance C of the ultrasonic transducer 206. If the ultrasonic transducer capacitance C is not known, it can be measured as explained above, but it could also be known.

However, based on this first harmonic trigonometric coefficients $a_{1,u}$ and $b_{1,u}$ and the known capacitance C of the transducer 206, a capacitance current component of the overall current $i_m$ can be calculated and subtracted from the overall current $i_m$.

This calculation is done in the modification block 216. The modification block 216 therefore receives the first harmonic trigonometric coefficients $a_{1,u}$ and $b_{1,u}$ of the measured instrument voltage $u_m$ and receives the first harmonic Fourier trigonometric coefficients $a_{1,i}$ and $b_{1,I}$ of the current signal and also the capacitance C of the ultrasonic transducer 206. One possibility to clear the overall current $i_m$ from its capacitance current component is to use these first harmonic trigonometric coefficients. The underlying idea is that only the first harmonic of the overall current and also only the first harmonic of the instrument voltage is considered, as only that is relevant for setting the signal frequency f and only that is relevant for considering the capacitance current component. Accordingly, not the whole overall current $i_m$ is used but only the first harmonic and accordingly it is possible to only consider the corresponding coefficients of the first harmonic of the overall current as well as of the instrument voltage. This is done in the modification block 216 and it was also found that it is possible to subtract the capacitance current component by just modifying the first harmonic Fourier trigonometric coefficients $a_{1,i}$ and $b_{1,I}$ into $a'_{1,i}$ and $b'_{1,i}$.

The overall current $i_m$ is thus cleared from the capacitance current component by subtracting it and the result is the remaining main current component. The first harmonic of this main current component is thus described by the modified first harmonic Fourier trigonometric coefficients $a'_{1,i}$ and $b'_{1,i}$.

Accordingly, for calculating the phase shift between the main current component and the instrument voltage, the corresponding coefficients $a'_{1,i}$ and $b'_{1,i}$, as well as $a_{1,u}$ and $b_{1,u}$ are given into the phase shift calculation block 218. This phase shift calculation block 218 thus calculates the phase shift $\varphi$ and this calculated phase shift $\varphi$ is inputted in the frequency adjustment block 220. The frequency adjustment block 220 also receives the signal frequency f which is the actual signal frequency and adds a small offset $\Delta f$ or subtracts such small offset $\Delta f$ or leaves the signal frequency f unchanged if the calculated phase shift $\varphi$ is zero or below a relevance threshold.

Accordingly, the result of the frequency adjustment block 220 is the adjusted frequency $f_a$. This adjusted frequency $f_a$ is given to the amplifier 204 and the amplifier 204 takes this adjusted frequency $f_a$ as the new signal frequency f. Accordingly, the amplifier 204 generates and outputs the overall current $i_m$ having this signal frequency f. Accordingly, this overall current $i_m$ is given into the electrosurgical instrument 202 resulting in the instrument voltage $u_m$ as explained above. With these new values for the overall current $i_m$ and the instrument voltage $u_m$, the next control cycle can begin.

Figure 3 shows an equivalent circuit of an ultrasonic transducer showing the mechanical series resonant circuit given by a so-called mechanical capacitance CM, a mechanical inductance LM and a mechanical resistance RM. These three elements represent the electrical behavior of the ultrasonic transducer as far as it is depending on the mechanical behavior of the ultrasonic transducer.

In parallel, there is the electrical capacity CE which represents a capacitive behavior of this ultrasonic transducer which is not depending on the mechanical behavior of the ultrasonic transducer.

Accordingly, when in operation, this equivalent circuit 300 explains the components of the overall current $i_m$ which is driven into the ultrasonic transducer which is split into the capacitance current ic and the main current component i'. Accordingly, the equivalent circuit 300 also shows the instrument voltage $u_m$.

Figure 4 shows a diagram of two sin signals with different phase angles and thus having a phase shift with respect to each other. Both sin signals comprise white noise and for both sin signals the first harmonic is calculated and thus reconstructed using the first harmonic Fourier trigonometric coefficients $a_1$ and $b_1$. The resulting sin signals A and B are shown in Figure 4. Accordingly, these two signals A and B are given each by their first harmonic Fourier trigonometric coefficients $a_1$ and $b_1$ and these Fourier coefficients can be converted into the actual phase shift between signals A and B using the atan2 function as explained above and as will be further explained below.

Further aspects and explanations helpful to better understand the invention are given below.

It was found that the phase shift between the relevant signals, i.e. the voltage and the current, can be determined in the digital domain using a robust algorithm that does not require low-pass filtering as it automatically extracts the first harmonic of the signal. The idea is to calculate the first harmonic Fourier trigonometric coefficients $a_1$ and $b_1$ of the current and voltage according to:

$$a_1 = \frac{2}{N} \sum_{k=0}^{N-1} x[k] \cdot \cos\left(2\pi \frac{k}{N}\right)$$

$$b_1 = \frac{2}{N} \sum_{k=0}^{N-1} x[k] \cdot \sin\left(2\pi \frac{k}{N}\right)$$

Where $x[k]$ are the current/voltage samples, and $\sin\left(2\pi \frac{k}{N}\right)$ equals the reference signal that is used by the amplifier and created via a numerically controlled oscillation (NCO). The samples of the cos function can be efficiently created with the same NCO.

The relative phase shift of the signal can then be calculated as atan2($a_1$, $b_1$).

In total, four coefficients need to be calculated, two for the measured current $i_{meas}(t)$ and two for the measured voltage $u_{meas}(t)$, illustrated as follows :

$$i_{meas}(t) \quad \leftrightarrow \quad a_{1,i} \ , \ b_{1,i}$$

$$u_{meas}(t) \quad \leftrightarrow \quad a_{1,u} \ , \ b_{1,u}$$

The phase shift between the voltage and current is therefore atan2($a_{1,u}$, $b_{1,u}$) - atan2($a_{1,i}$, $b_{1,i}$). Either the two atan2 functions can be calculated separately or the atan2 difference identity may be used to reduce the calculation to a single atan2 function:

$$\text{atan2}\left(a_{1,u}, b_{1,u}\right) - \text{atan2}\left(a_{1,i}, b_{1,i}\right) = \text{atan2}\left(a_{1,u}b_{1,i} - a_{1,i}b_{1,u}, b_{1,u}b_{1,i} + a_{1,u}a_{1,i}\right)$$

Although the atan2 function can be calculated efficiently with digital logic using the CORDIC algorithm, the atan2 function can be reduced to a division by linearizing the function at $\varphi = 0$ as the phase locked loop (PLL) makes sure that the phase shift is always close to 0:

$$\text{atan2}(y, x) = \varphi \approx \frac{y}{x} \quad \bigg| \varphi \approx 0$$

Typically, an ultrasonic inverter implements voltage, current and power limits. As a side-catch, the calculated Fourier coefficients can be directly converted into RMS current/voltage and real power according to:

$$i_{RMS} = \sqrt{\frac{a_{1,i}{}^2 + b_{1,i}{}^2}{2}}$$

$$u_{RMS} = \sqrt{\frac{a_{1,u}{}^2 + b_{1,u}{}^2}{2}}$$

$$P = a_{1,i} a_{1,u} + b_{1,i} b_{1,u}$$

Additional improvements are suggested as follows:

The following improvements are basically independent of the phase-shift detection algorithm but can be implemented very efficiently when using the Fourier coefficient based algorithm described above.

Compensation of electrical transducer capacitance can be done as follows.

The current through the instrument consists of a main current component, and can be denoted as "mechanical" part, and a capacitive component and can be denoted as "capacitive" part or capacitive current flowing through the electrical capacitance. This capacitive current should be compensated for two reasons. First, the phase shift must be calculated between the voltage and the "mechanical" current. Second, the ultrasonic amplifier usually operates in a constant "mechanical" current mode.

The current can be compensated by subtracting the current through the capacitance which equals the derivative of the measured voltage times the electrical capacitance of the instrument. That is expressed in the following formula in which i'(t) is the main current component, i.e. the current without the capacitive part, $i_{meas}(t)$ is the overall current, $u_{meas}(t)$ is the instrument voltage and $i_c(t)$ is the capacitive current component.

$$i'(t) = i_{meas}(t) - i_c(t) = i_{meas}(t) - CE \cdot \frac{d\,u_{meas}(t)}{dt}$$

Calculating the derivative in the time domain and thus differentiation in the time domain is very sensitive to noise. Instead, the Fourier coefficients can be used to differentiate in the frequency domain:

If the first harmonic Fourier coefficients of $i_{meas}(t)$ / $u_{meas}(t)$ are given by $a_{1,i}$, $b_{1,i}$ and $a_{1,u}$, $b_{1,u}$, the current and voltage can be described by the formulas describing the Fourier Series:

$$i_{meas}(t) = a_{1,i} \cdot \cos(\omega_0 t) + b_{1,i} \cdot \sin(\omega_0 t) + residual$$

$$u_{meas}(t) = a_{1,u} \cdot \cos(\omega_0 t) + b_{1,u} \cdot \sin(\omega_0 t) + residual$$

The residual represents the further harmonics and as only the first harmonic is considered, the residuals can be neglected. Accordingly said formulas can be written as:

$$i_{meas}(t) \approx a_{1,i} \cdot \cos(\omega_0 t) + b_{1,i} \cdot \sin(\omega_0 t)$$

$$u_{meas}(t) \approx a_{1,u} \cdot \cos(\omega_0 t) + b_{1,u} \cdot \sin(\omega_0 t)$$

Based on that the coefficients are considered below implying that the above formulas are underlying the consideration. Thus the relationship can be symbolized and explained as follows, wherein the double arrows indicate that the coefficients on the right side shall be meant to be used in the corresponding formula as given above:

$$i_{meas}(t) \quad \leftrightarrow \quad a_{1,i} \ , \ b_{1,i}$$

$$u_{meas}(t) \quad \leftrightarrow \quad a_{1,u} \ , \ b_{1,u}$$

Based on that the Fourier coefficients of the voltage derivative can be calculated using the properties of the Fourier Series (s. above):

$$\frac{d\,u_{meas}(t)}{dt} \quad \leftrightarrow \quad \omega_0 b_{1,u} \ , \ -\omega_0 a_{1,u}$$

$$i'(t) = i_{meas}(t) - CE \cdot \frac{d\,u_{meas}(t)}{dt} \quad \leftrightarrow \quad a_{1,i} - CE\omega_0 b_{1,u} \ , \ b_{1,i} + CE\omega_0 a_{1,u}$$

That is:

$$i'(t) \approx \left(a_{1,i} - CE \cdot \omega_0 \cdot b_{1,u}\right)\cos(\omega_0 t) + \left(b_{1,i} + CE \cdot \omega_0 \cdot a_{1,u}\right)\sin(\omega_0 t)$$

Differentiation in the time domain has become a multiplication in the frequency domain. The last formula is just for clarification as it might be enough to just use the changed coefficients for further consideration.

The compensation of the capacitive current component described above thus requires the factor $CE\omega_0$ ($CE^{*}\omega_0$) which equals the magnitude of the electrical capacitor admittance at the resonance frequency. When the capacitance is unknown, it can be determined by driving the instrument out of resonance and calculating

$$CE\omega_0 = \frac{I_{meas,rms}}{U_{meas,rms}} \cdot \frac{\omega_0}{\omega_{meas}}$$

i.e., the measured admittance of the instrumented at the measurement frequency, scaled by the ratio between the actual resonance frequency and the measurement frequency.

A further suggestion is to use virtual instrument load.

When the mechanical load on the instrument is low, the amplitude of the voltage can become very low. This can make it difficult to determine the phase shift between voltage and current. To make the algorithm more robust, a virtual resistance can be added to the instrument by adding a virtual voltage to the measured voltage which is proportional to the (compensated) current. Accordingly, a modified voltage u'(t) results considering a virtual Resistance $R_{virt}$:

$$u'(t) = u_{meas}(t) + R_{virt} \cdot i'(t)$$

Again, this can be realized in the frequency domain very easily. Using the definitions from above:

$$i_{meas}(t) \quad \leftrightarrow \quad a_{1,i} \ , \ b_{1,i}$$

$$u_{meas}(t) \quad \leftrightarrow \quad a_{1,u} \ , \ b_{1,u}$$

$$i'(t) \quad \leftrightarrow \quad a_{1,i} - CE\omega_0 b_{1,u} \ , \ b_{1,i} + CE\omega_0 a_{1,u}$$

Yields the Fourier coefficients for the modified (compensated) voltage:

$$u'(t) \quad \leftrightarrow \quad a_{1,u} + R_{virt}\left(a_{1,i} - CE\omega_0 b_{1,u}\right) \ , \ b_{1,u} + R_{virt}\left(b_{1,i} + CE\omega_0 a_{1,u}\right)$$

That is:

$$u'(t) \approx \left(a_{1,u} + R_{virt}\left(a_{1,i} - CE \cdot \omega_0 \cdot b_{1,u}\right)\right) \cdot \cos \omega_0 t + \left(b_{1,u} + \left(b_{1,i} + CE \cdot \omega_0 \cdot a_{1,u}\right)\right) \sin \omega_0 t$$

The approximately equal indicates that the further harmonics are neglected.

**Claims**

1. Method for controlling an ultrasonic transducer (206) of an electrosurgical instrument (102, 202),

   - the ultrasonic transducer (206) is driven by an alternating electrical overall current signal ($i_m$), having a signal frequency (f),
   - driving the overall current signal ($i_m$) results in an alternating electrical instrument voltage signal ($u_m$) of the transducer (206), depending on a mechanical oscillation of the transducer (206)
   - the instrument voltage signal ($u_m$) having a transducer phase shift with respect to a main current component of the overall current signal ($i_m$) depending on the mechanical oscillations of the transducer,
   - the ultrasonic transducer (206) is having a fluctuating mechanical resonance frequency,
   - an amplifier (204) is controlled to provide the electrical overall current signal ($i_m$) and adjust the signal frequency (f) towards the resonance frequency depending on the transducer phase shift, and
   - for determining the transducer phase shift,
   - calculating signal coefficients for the main current component and the instrument voltage signal ($u_m$), the signal coefficients being representative for a phase relationship of the main current component or the instrument voltage signal respectively with respect to a common reference signal, and
   - calculating from the signal coefficients the transducer phase shift.

2. Method according to claim 1, **characterized in that**

   - depending on the calculated transducer phase shift
   - adjusting the signal frequency (f) to match the mechanical resonance frequency or to get closer to it, in particular
   - determining whether the signal frequency (f) is above or below the resonance frequency and
   - raising or reducing the signal frequency (f) accordingly to get closer to the resonance frequency, or
   - calculating an actual value of the mechanical resonance frequency depending on the calculated transducer phase shift and
   - setting the signal frequency (f) depending on the calculated mechanical resonance frequency.

3. Method according to claim 1 or 2, **characterized in that**

   - the calculating of the signal coefficients is based on samples of the current signal and samples of the main voltage signal, in particular samples of the measured current signal and samples of the main voltage signal determined from the measured alternating electrical voltage of the transducer (206).

4. Method according to any of the preceding claims, **characterized in that**

   - the signal coefficients characterize the first harmonic of the corresponding signal.

5. Method according to any of the preceding claims, **characterized in that**

   - for each signal, in particular for the main current component and the instrument voltage signal ($u_m$) each, the signal coefficients are the first harmonic Fourier trigonometric coefficients $a_1$ and $b_1$ calculated according to the formula:

$$a_1 = \frac{2}{N} \sum_{k=0}^{N-1} x[k] * \cos\left(2\pi \frac{k}{N}\right)$$

$$b_1 = \frac{2}{N} \sum_{k=0}^{N-1} x[k] * \sin\left(2\pi \frac{k}{N}\right)$$

wherein
- x[k] are samples of the corresponding signal and
- N is the number of samples per signal, and wherein
- the samples are taken over a predetermined time interval and/or a time interval of one period corresponding to a nominal value of the resonance frequency.

6. Method according to any of the preceding claims, **characterized in that**

- for the signal coefficients for each signal being the first harmonic Fourier trigonometric coefficients $a_1$ and $b_1$
- the absolute phase shift $\varphi_a$, for each signal is calculated based on the formula

$$\varphi_a = atan2(a_1, b_1)$$

- and the transducer phase shift is calculated as a difference of the absolute phase shifts of both signals.

7. Method according to any of the preceding claims, **characterized in that**

- for the signal coefficients for each signal being the first harmonic Fourier trigonometric coefficients $a_1$ and $b_1$
- the transducer phase shift is calculated by an approximation such that the transducer phase shift $\varphi_t$ is calculated as the difference of the quotient $a_1/b_1$ of both signals, in particular $\varphi_t$ is calculated using the formula

$$\varphi_t = a_{1,i}/b_{1,i} - a_{1,u}/b_{1,u}$$

with

$a_{1,i}$ and $b_{1,i}$ being the first harmonic Fourier trigonometric coefficients $a_1$ and $b_1$ of the main current component and
$a_{1,u}$ and $b_{1,u}$ being the first harmonic Fourier trigonometric coefficients $a_1$ and of the instrument voltage signal ($u_m$).

8. Method according to any of the preceding claims, **characterized in that**

- calculating the transducer phase shift and/or adjusting the signal frequency (f) to match the mechanical resonance frequency orto get closer to it, is repeated regularly for tracking changes of the mechanical resonance frequency, and in particular
- the calculating or adjusting is repeated with a predetermined repetition frequency being at least one repetition per 10 signal periods, preferably at least one repetition per 2 signal periods, in particular one repetition per signal period.

9. Method according to any of the preceding claims, **characterized in that**

- the signal coefficients, in particular the signal coefficients for each signal being the first harmonic Fourier trigonometric coefficients $a_1$ and $b_1$, are used to calculate
- an rms-value of the current signal, and/or
- an rms-value of the voltage signal and/or
- a real power value of both signals, wherein in particular
- each rms-value and/or the real power value of both signals is calculated based on the first harmonic of the current signal and/or the voltage signal.

10. Method according to any of the preceding claims, **characterized in that**

- the overall current signal ($i_m$), having a main current component and a capacitive current component, the capacitive current component, depending on a capacitance (C) of the transducer (206), and
- the main current component is calculated by subtracting the capacitive current component from the overall current signal ($i_m$), in particular from a measured alternating electrical current signal of the transducer (206), and/or
- first harmonic Fourier trigonometric coefficients $a_1$ and $b_1$ are used for calculating the capacitive current signal, wherein in particular
- the capacitive current component ic is calculated for the capacitance (C) and the frequency $\omega$ using the formula:

$$i_C = C*\omega*a_1*\cos(\omega*t) \text{ or } i_C = -C*\omega*b_1*\sin(\omega*t).$$

11. Method according to any of the preceding claims, **characterized in that**

- for calculating a or the transducer capacitance (C),
- the overall current signal ($i_m$) is generated having a test frequency being different to a or the nominal value of the resonance frequency at least by a minimum frequency difference, resulting in an instrument voltage signal ($u_m$),
- the signal coefficients are calculated based on the overall current signal ($i_m$) and the resulting instrument voltage signal ($u_m$) having the test frequency, and
- the transducer capacitance (C) is calculated based on these signal coefficients, wherein in particular
- the minimum frequency difference is at least 10kHz, in particular at least 20 kHz.

12. Method according to any of the preceding claims, **characterized in that**

- for calculating the signal coefficients, in particular for calculating the signal coefficients of the instrument voltage signal ($u_m$),
- the instrument voltage signal ($u_m$) is modified into a modified voltage signal by adding a virtual voltage signal, wherein
- the virtual voltage signal is calculated by multiplying the main current component with a predetermined virtual resistance, and in particular
- as the main current component a previously calculated main current component is used and,
- the previously calculated main current signal is calculated based on the signal coefficients previously calculated for the voltage signal and for the main current signal and/or
- the modified voltage signal is calculated directly using the signal coefficients previously calculated for the voltage signal and for the main current signal and/or
- coefficients of the modified voltage signal are calculated directly using signal coefficients previously calculated for the voltage signal and for the main current signal and using the predetermined virtual resistance.

13. Electrosurgical generator (100) for controlling an ultrasonic transducer (206) of an electro surgical instrument (102, 202) adapted to execute a method for controlling an ultrasonic transducer (206) according to any of the preceding claims.

14. Electro surgical installation comprising

- an electro surgical generator (100) according to claim 13 and
- an electro surgical instrument (102, 202) having an ultrasonic transducer (206) connected to the electro surgical generator (100).

Fig. 1

Fig. 2

Fig. 3

Fig. 4

Europäisches
Patentamt

European
Patent Office

Office européen
des brevets

**EUROPEAN SEARCH REPORT**

Application Number

EP 24 18 9201

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 115 040 200 A (YINUOKANG MEDICAL TECH SUZHOU CO LTD) 13 September 2022 (2022-09-13) | 1-4,8, 13,14 | INV. A61B17/32 A61B18/12 |
| A | * the whole document * | 5-7,9-12 | |
| X | CN 115 844 497 A (SHENZHEN SUIGSCI MEDICAL TECH CO LTD) 28 March 2023 (2023-03-28) | 1-3,8, 13,14 | |
| A | * the whole document * | 5-7,9-12 | |
| A | EP 3 505 097 A1 (ETHICON LLC [PR]) 3 July 2019 (2019-07-03) * paragraph [0218] - paragraph [0263] * | 1-14 | |
| A | WO 2020/112688 A1 (ULTHERA INC [US]) 4 June 2020 (2020-06-04) * paragraph [0115] - paragraph [0145] * | 1-14 | |

TECHNICAL FIELDS
SEARCHED        (IPC)

A61B

The present search report has been drawn up for all claims

| Place of search | Date of completion of the search | Examiner |
|---|---|---|
| The Hague | 5 December 2024 | Emirdag, Eda |

EPO FORM 1503 03.82 (P04C01)

## ANNEX TO THE EUROPEAN SEARCH REPORT
## ON EUROPEAN PATENT APPLICATION NO.

EP 24 18 9201

This annex lists the patent family members relating to the patent documents cited in the above-mentioned European search report.
The members are as contained in the European Patent Office EDP file on
The European Patent Office is in no way liable for these particulars which are merely given for the purpose of information.

05-12-2024

| Patent document cited in search report | | Publication date | Patent family member(s) | | Publication date |
|---|---|---|---|---|---|
| CN 115040200 | A | 13-09-2022 | CN | 115040200 A | 13-09-2022 |
| | | | WO | 2023221379 A1 | 23-11-2023 |
| CN 115844497 | A | 28-03-2023 | NONE | | |
| EP 3505097 | A1 | 03-07-2019 | EP | 3505097 A1 | 03-07-2019 |
| | | | EP | 3505099 A1 | 03-07-2019 |
| | | | EP | 3505100 A1 | 03-07-2019 |
| | | | EP | 3505108 A1 | 03-07-2019 |
| | | | US | 2019201037 A1 | 04-07-2019 |
| | | | US | 2019201038 A1 | 04-07-2019 |
| | | | US | 2019201042 A1 | 04-07-2019 |
| | | | US | 2021153889 A1 | 27-05-2021 |
| | | | US | 2021169516 A1 | 10-06-2021 |
| | | | US | 2021177452 A1 | 17-06-2021 |
| | | | US | 2021212719 A1 | 15-07-2021 |
| WO 2020112688 | A1 | 04-06-2020 | AU | 2019385902 A1 | 20-05-2021 |
| | | | BR | 112021006999 A2 | 20-07-2021 |
| | | | CA | 3116332 A1 | 04-06-2020 |
| | | | CN | 113260414 A | 13-08-2021 |
| | | | EP | 3886981 A1 | 06-10-2021 |
| | | | IL | 282095 A | 31-05-2021 |
| | | | JP | 2022513577 A | 09-02-2022 |
| | | | KR | 20210110578 A | 08-09-2021 |
| | | | SG | 11202102775V A | 29-04-2021 |
| | | | TW | 202023646 A | 01-07-2020 |
| | | | TW | 202434335 A | 01-09-2024 |
| | | | US | 2022023670 A1 | 27-01-2022 |
| | | | WO | 2020112688 A1 | 04-06-2020 |

EPO FORM P0459

For more details about this annex : see Official Journal of the European Patent Office, No. 12/82